# EUROPÄISCHE PATENTANMELDUNG

(11) **EP 0 700 988 A2**
(43) Veröffentlichungstag der Anmeldung: **13.03.1996**
(21) Anmeldenummer: 95110692.1
(22) Anmeldetag: 08.07.1995
(51) Int. Cl.: C11D 7/50, C23G 5/032, G03G 11/00

(54) **Anwendung und Zusammensetzungen mit Difluormethoxy-2,2,2-trifluorethan**

(30) Priorität: 15.07.1994 DE 4425066
(71) Anmelder: Solvay Fluor und Derivate GmbH, D-30173 Hannover (DE)
(72) Erfinder: Buchwald, Hans, Dr., D-30952 Ronnenberg (DE); Hellmann, Joachim, D-30171 Hannover (DE); Raszkowski, Boleslaus, D-31719 Wiedensahl (DE)
(74) Vertreter: Lauer, Dieter, Dr.

(57) **Zusammenfassung**

Die vorliegende Erfindung bezieht sich auf neue Anwendungen von Difluormethoxy-2,2,2-trifluorethan (E245) und von Zusammensetzungen, die außer Difluormethoxy-2,2,2-trifluorethan ein weiteres Lösungsmittel (Colösungsmittel) enthalten. Die Erfindung bezieht sich weiterhin auf neue Zusammensetzungen von E245 im Gemisch mit einigen dieser Lösungsmittel. Als bevorzugte Anwendung wird ein Verfahren zur Fixierung von auf einem Aufzeichnungsmaterial aufgebrachten Toner in der Fixiereinrichtung von Druck- und Kopiergeräten mittels Fixierdampf auf Basis von E245 beschrieben; bei diesem Verfahren gelangt E245 als solches oder im Gemisch mit einem Colösungsmittel zur Anwendung.

## Beschreibung

Die vorliegende Erfindung bezieht sich auf neue Anwendungen von Difluormethoxy-2,2,2-trifluorethan (E245) und von Zusammensetzungen, die außer Difluormethoxy-2,2,2-trifluorethan ein weiteres Lösungsmittel (Colösungsmittel) enthalten. Die Erfindung bezieht sich weiterhin auf neue Zusammensetzungen von E245 im Gemisch mit einigen dieser Lösungsmittel. Als bevorzugte Anwendung wird ein Verfahren zur Fixierung von auf einem Aufzeichnungsmaterial aufgebrachten Toner in der Fixiereinrichtung von Druck- und Kopiergeräten mittels Fixierdampf auf Basis von E245 beschrieben; bei diesem Verfahren gelangt E245 als solches oder im Gemisch mit einem Colösungsmittel zur Anwendung.

Beim Druck- und Kopiervorgang wird durch Ablichten der Druck- bzw. Kopiervorlage zunächst auf einer Fotoleitertrommel ein Ladungsbild (Negativbild) erzeugt, welches in einer nachfolgenden Entwicklerstation entwickelt wird, d. h. das Ladungsbild wird mit Toner versehen. Die entwickelten Ladungsbilder auf der Fotoleitertrommel gelangen anschließend in eine Umdruckstation. Durch ein in der Umdruckstation bestehendes elektrostatisches Feld wird das Tonerbild auf einen der Umdruckstation zugeführten Aufzeichnungsträger, z. B. eine Papierbahn oder auch ein Einzelblatt, herübergezogen. Das Tonerbild haftet nunmehr, allerdings verwischbar auf der Oberfläche des Aufzeichnungsträgers. Der mit dem Tonerbild (Positivbild) versehene Aufzeichnungsträger wird hiernachdurch eine Fixiereinrichtung hindurchgeführt. In dieser Fixiereinrichtung wird ein zum Fixieren des Toners erforderlicher Lösungsmitteldampf erzeugt. Dazu ist das Lösungsmittel am Boden des Gehäuses der Fixiervorrichtung angeordnet. Der Boden der Fixiervorrichtung wird mittels einer Heizvorrichtung erwärmt, so daß das Lösungsmittel vom Boden verdampft. Der hierdurch erzeugte Lösungsmitteldampf wirkt auf den Aufzeichnungsträger und insbesondere auf den darauf aufgebrachten Toner ein. Bei diesem Verfahren, der sogenannten Kaltfixierung, sorgt also der Lösungsmitteldampf für einen innigen Kontakt zwischen Toner und Aufzeichnungsmaterial. Der Toner wird hierbei angelöst, so daß dieser in den Aufzeichnungsträger eindringen kann. Nach diesem Fixiervorgang werden die Aufzeichnungsträger durch Trocknen von ggf. noch anhaftenden Resten des Lösungsmitteldampfes befreit und man erhält ein trockenes, gut haftendes und nicht verwischbares Tonerbild auf dem Aufzeichnungsträger.

An Lösungsmittel für die Fixierung von Toner werden besondere Anforderungen gestellt. Solche Lösungsmittel sollten einen relativ niedrigen Siedepunkt aufweisen und nicht entflammbar und weitgehend untoxisch sein, sowie günstige Löseeigenschaften für z. B. auf einem Aufzeichnungsmaterial aufgebrachte und durch Anlösen zu fixierende Tonerpartikel aufweisen. Diese Anforderungen lassen sich in der Regel nicht mit nur einem einzigen Lösungsmittel erfüllen. Daher kommen in der Praxis eine Vielzahl von Lösungsmittelgemischen mit mehr oder weniger unterschiedlichen Zusammensetzungen zur Anwendung. Im Stand der Technik ist es daher (z. B. im Bereich der industriellen Reinigungsverfahren oder bei der Dampfentfettung) bekannt, neben reinen chlorierten und/oder fluorierten Kohlenwasserstoffen auch Gemische von Fluorchlorkohlenwasserstoffen (als Hauptlösungsmittel) mit einem Colösungsmittel einzusetzen. Solche Gemische können sowohl nichtazeotrop als auch azeotrop bzw. azeotropartig sein. Der Begriff "azeotropartig" bedeutet dabei, daß die Gemische über einen größeren Mischungs- bzw. Konzentrationsbereich im wesentlichen konstant sieden (Änderung der Siedetemperatur um nicht mehr als 5 °C). Solche azeotropartigen Gemische verhalten sich daher im praktischen Einsatz ähnlich wie Azeotrope. Als Azeotrope werden solche Lösungsmittelgemische angesehen, die einen konstanten Siedepunkt (maximale Schwankung +/- 0,5 °C) besitzen und die beim Sieden in der Dampfphase die Lösungsmittelkomponenten des Gemisches in derselben relativen Zusammensetzung wie in der Flüssigphase enthalten. Lösungsmittelgemische, die sich für die Anwendung in Fixiereinrichtungen von Druck- und Kopiergeräten eignen, bestehen in der Regel aus wenigstens zwei Lösungsmittelkomponenten, von denen wenigstens eine Komponente toneranlösende Eigenschaften aufweisen muß. Da diese Lösungsmittelgemische in modernen Fixiereinrichtungen im Dampfzustand auf den Toner einwirken, wird von praxisgerechten Lösungsmittelgemischen eine möglichst gleichmäßige Verdampfung verlangt, d. h. ein Verdampfungsverhalten ohne Auftrennung in die Komponenten bzw. wenigstens ohne größere Verschiebungen der Mengenverhältnisse der Komponenten in der Dampfphase relativ zur Flüssigphase. Zweckmäßig besitzt das in der Fixiereinrichtung eingesetzte Lösungsmittelgemisch somit wenigstens azeotropartige, insbesondere aber azeotrope Siedeeigenschaften, um auch über längere Anwendungszeiträume eine gleichbleibend gute Fixierung des Toners auf dem Aufzeichnungsmaterial gewährleisten zu können.

Die Anwendung von Lösungsmitteln in der Fixiervorrichtung von Druck- oder Kopiergeräten ist beispielsweise bereits in der deutschen Patentschrift DE 28 35 284 beschrieben; es werden dort azeotrope Gemische aus Trichlortrifluorethan (R113) und Aceton vorgeschlagen. In dieser Schrift finden sich auch weitere Einzelheiten zum Ablauf der Tonerfixierung im Rahmen des Druck- und Kopiervorganges. Ein weiteres Verfahren unter Anwendung von teilhalogenierten Fluorchlorkohlenwasserstoffen ist in der WO 93/10485 beschrieben; als Hauptlösungsmittel werden 2,2-Dichlor-1,1,1-trifluorethan (R123) und 1,1-Dichlor-1-fluorethan (R141b) vorgeschlagen. Nachteilig ist jedoch, daß einerseits das vorgeschlagene R123 toxikologisch unter Umständen nicht ganz unbedenklich ist und daß andererseits R141b ggf. Materialunverträglichkeiten aufweist, die den Einsatz einschränken können.

Zwar wurden im Stand der Technik bereits einige Anstrengungen unternommen, um für verschiedene Anwendungsgebiete alternative Zusammensetzungen mit den gewünschten Eigenschaften für den Ersatz der bisher verwendeten, vollhalogenierten Kohlenwasserstoffe aufzufinden, doch besteht aufgrund von anwendungstechnischen, toxikologischen und die Umwelt beeinflussenden Eigenschaften immer noch ein Verbesserungsbedarf. Beispielsweise enthalten bekannte Lösungsmittelgemische größere Anteile an toxikologisch und unter Umweltgesichtspunkten sowie ggf. unter Sicherheitsaspekten (niedriger Flammpunkt) bedenklichen Lösungsmitteln. Für eine Reihe von Lösungsmittelbestandteilen ist aufgrund ihrer die Umwelt beeinflussenden Eigenschaften ein Ersatz durch andere, für die jeweiligen Anwendungszwecke mindestens gleichermaßen gut geeignete Lösungsmittel wünschenswert.

Es bestand daher die Aufgabe, Lösungsmittel bzw. Lösungsmittelgemische aufzufinden, die insbesondere für die Verwendung als Tonerlösungsmittel in der Fixiereinrichtung von Druck- und Kopiergeräten gut geeignet sind.

Zur Lösung dieser Aufgabe schlägt die Erfindung die Verwendung von Difluormethoxy-2,2,2-trifluorethan als Lösungsmittel für die Fixierung von auf einem Aufzeichnungsmaterial aufgebrachten Tonerpartikeln, beispielsweise in der Fixiereinrichtung von Druck- und Kopiergeräten sowie insbesondere Laserdruckern, vor. Difluormethoxy-2,2,2-trifluorethan, welches allgemein auch als E245 bezeichnet wird, ist bereits bekannt und kann z. B. durch Umsetzung von Trifluorethanol mit Chlordifluormethan, wie diese Umsetzung beispielsweise im US-Patent 3,761,524 beschrieben ist, hergestellt werden. Es wurde erfindungsgemäß gefunden, daß E245 im Hinblick auf die vorstehende Verwendung und die weiter unten noch beschriebenen weiteren Anwendungen günstige Lösungsmitteleigenschaften aufweist. E245 zeigt günstiges Quellverhalten von verschiedenen Kunststoffen und auch von Elastomeren. Organische Stoffe polarer Natur sind in E245 gut löslich. E245 eignet sich darüber hinaus aber auch für Anwendungen, bei denen der Zusatz unpolarer Additive erwünscht ist; in diesem Fall kann die Löslichkeit der unpolaren Additive durch geeignete Lösungsvermittler verbessert werden. Ferner ist E245 mit vielen Kunststoffen gut verträglich, die durch die bisher im Stand der Technik verwendeten voll- und teilhalogenierten Fluorchlorkohlenwasserstoffe angegriffen werden. Darüber hinaus ist E245 nicht brennbar und weist keinen Flammpunkt auf. Der Siedepunkt von E245 beträgt bei Atmosphärendruck 29 °C.

Das erfindungsgemäß verwendete Difluormethory-2,2,2-trifluorethan eignet sich daher bereits als solches zur Fixierung, insbesondere zur Fixierung polarer Tonerpartikel; aber auch mit unpolaren Tonerpartikeln können unter Verwendung von Difluor-methoxy-2,2,2-trifluorethan als Lösungsmittel brauchbare Fixierungsergebnisse erzielt werden. Die fixierenden Eigenschaften können durch Zusatz von Colösungsmitteln sowie gegebenenfalls auch durch Zusatz von Lösungsvermittlern (z. B. im Falle unpolarer Additive) gegenüber der alleinigen Verwendung von E245 verbessert werden. In einer bevorzugten Ausgestaltung der Erfindung wird daher E245 als Hauptlösungsmittel in Kombination mit einem Colösungsmittel verwendet. Solche Colösungsmittel können insbesondere die in üblichen Fixierungslösungsmitteln enthaltenen Colösungsmittel sein. E245 eignet sich insbesondere im Gemisch mit Colösungsmitteln auch für weitere Anwendungen, z. B. Reinigungsanwendungen. Zweckmäßig sind die Colösungsmittel für die Fixierungsanwendung ausgewählt aus der Gruppe der niederen Alkohole, Ketone, Ester und gegebenenfalls der chlorierten Kohlenwasserstoffe (CKW). Bei Reinigungsanwendungen kommen zusätzlich zu den vorgenannten Colösungsmitteln auch Glykole in Frage.

Beispiele für als Colösungsmittel geeignete niedere Alkohole sind insbesondere C1- bis C3-Alkohole, z. B. Methanol, Ethanol, Propanol, Isopropanol. Die Gruppe der Ketone umfaßt insbesondere die C3- bis C6-Ketone; beispielsweise Aceton, Methylethylketon, Methylisobutylketon; bevorzugtes Keton ist Aceton. Für die Gruppe der Ester sind insbesondere die Ester von C2- bis C3-Carbonsäuren mit niederen Alkoholen (C1- bis C3-Alkohole) zu nennen; zweckmäßig sind z. B. Ester der Essigsäure oder der Propionsäure mit den C1- bis C2-Alkoholen Methanol oder Ethanol. Bevorzugte Ester sind z. B. Methylacetat und Ethylacetat, aber auch Methylpropionat. Die weitere Gruppe der Colösungsmittel, d. h. die chlorierten Kohlenwasserstoffe, sind niedermolekulare, d. h. C1- bis C2-Kohlenwasserstoffe, die durch ein oder mehrere Chloratome substituiert sind. Gegebenenfalls können die chlorierten Kohlenwasserstoffe zusätzlich auch Fluoratome tragen. Bevorzugte chlorierte Kohlenwasserstoffe (CKW) für die erfindungsgemäße Verwendung als Colösungsmittel für E245 sind Methylenchlorid und Trichlorethylen. Die Gruppe der Glykole (nur für Reinigungsanwendungen) umfaßt neben der Stammverbindung (Ethylenglykol) die gattungsmäßigen Diole, deren Hydroxylgruppen vicinal angeordnet sind (1,2-Diole) sowie auch solche Verbindungen mit nicht-benachbarten OH-Gruppen als auch solche Verbindungen, in denen eine der beiden OH-Gruppen durch einen C1- bis C4-Alkylrest verethert ist. Die gemäß der Erfindung eingesetzten Glykole umfassen zwei bis zehn C-Atome. Beispiele für erfindungsgemäß verwendbare Glykole sind Ethylenglykol, Propylenglykole und Butylenglykole und insbesondere Butylglykol (2-Butoxy-ethanol).

Bei der erfindungsgemäßen Verwendung binärer Zusammensetzungen aus E245 als Hauptlösungsmittel und aus niederen Alkoholen, Ketonen, Estern und CKW als Colösungsmittel zur Fixierung von Tonerpartikeln sind solche binären Zusammensetzungen bevorzugt, die zumindest azeotropartige, insbesondere aber azeotrope Eigenschaften aufweisen. Im allgemeinen wird dann das Colösungsmittel in einer Menge von 0,2 bis 5,0 Gew.-%, insbesondere 0,3 bis 5,0 Gew.-% und das Hauptlösungsmittel E245 demgemäß in einer Menge von 99,8 bis 95,0 Gew.-% bzw. 99,7 bis 95,0 Gew.-% enthalten sein. In azeotropen binären Zusammensetzungen wird die Menge des Colösungsmittels je nach Art zwischen 0,3 bis 3,0 Gew.-% und die Menge von E245 demgemäß 99,7 bis 97,0 Gew.-% betragen. Die Summe der Bestandteile addiert sich hierbei jeweils auf 100 Gew.-%. Solche azeotropartigen bzw. azeotropen Zusammensetzungen können durch Destillationsversuche mit einer Mischung des Haupt- und Colösungsmittels und nachfolgender Analyse der jeweiligen Zusammensetzung von Dampf- und Flüssigphase ermittelt werden.

Binäre azeotropartige Zusammensetzungen aus E245 und niederen Alkoholen sind bereits in der europäischen Patentanmeldung 0 600 538 A1 für die Reinigung elektronischer Bauteile und für die Entfettung von Metallteilen beschrieben; auf den Inhalt der EP 0 600 538 A1 wird daher in Hinblick auf die Gewinnung von azeotropartigen bzw. azeotropen Zusammensetzungen aus E245 und niederen Alkoholen, sowie auf die hierzu offenbarten Mengenbereiche der beiden Komponenten ausdrücklich Bezug genommen. Beispielsweise enthält eine dort angebegene azeotrope Zusammensetzung etwa 97 Gew.-% E245 und etwa 3 Gew.-% Methanol; der Siedepunkt beträgt bei Atmosphärendruck (1 bar) etwa 29,3 °C. Eine weitere azeotrope Zusammensetzung enthält 99,7 Gew.-% E245 und 0,3 Gew.-% Ethanol; der Siedepunkt beträgt 74,9 °C bei 4 bar.

Die erfindungsgemäß zur Tonerfixierung verwendeten binären Zusammensetzungen aus E245 und den Colösungsmitteln aus der Gruppe der Ketone, Ester, chlorierten Kohlenwasserstoffe sind dagegen bisher im Stand der Technik nicht beschrieben. Es wurde hierbei auch gefunden, daß die Zusammensetzungen von E245 mit den vorstehenden Colösungsmitteln außer für die Verwendung zur Tonerfixierung auch für Lösungsmittel- bzw. Reinigungsanwendungen geeignet sind. Weiterhin wurde auch gefunden, daß Zusammensetzungen aus E245 mit einem Colösungsmittel aus der Gruppe der Glykole für Reinigungsanwendungen gut geeignet sind.

Die Erfindung betrifft daher auch die neuen Zusammensetzungen aus Difluormethoxy-2,2,2-trifluorethan und einem Colösungsmittel aus der Gruppe der Ketone, Ester, chlorierten Kohlenwasserstoffe und Glykole. Zweckmäßige Zusammensetzungen zeichnen sich hierbei durch einen Gehalt an 99,8 bis 50,0 Gew.-% Difluormethoxy-2,2,2-trifluorethan und 0,2 bis 50,0 Gew.-% des Colösungsmittels der Gruppe der Ketone, Ester, chlorierten Kohlenwasserstoffe und Glykole aus. Bevorzugte Zusammensetzungen für Reinigungsanwendungen enthalten 99,8 bis 90,0 Gew.-% Difluormethoxy-2,2,2-trifluorethan und 0,2 bis 10,0 Gew.-% eines Colösungsmittel aus der Gruppe der Ketone, Ester, chlorierten Kohlenwasserstoffe und Glykole. Diese Zusammensetzungen mit E245 zeigen im Falle der Colösungsmittel aus der Gruppe der Ketone, Ester und CKW ggf. bereits auch brauchbare Ergebnisse im Hinblick auf die Fixierung von Tonerpartikeln. Günstig sind hierbei Zusammensetzungen, die das Colösungsmittel aus der Gruppe der Ketone, Ester und CKW in einer Menge von 0,2 bis 5,0 Gew.-%, insbesondere 0,3 bis 5,0 Gew.-%, und das Hauptlösungsmittel E245 demgemäß in einer Menge von 99,8 bis 95,0 Gew.-% bzw. 99,7 bis 95,0 Gew.-% enthalten. Bevorzugt liegt das Colösungsmittel aus der Gruppe Ketone, Ester und CKW in einer Menge von 0,3 bis 5,0 Gew.-% vor; die Menge des E245 beträgt dann 99,7 bis 95,0 Gew.-%. Die Summe der Bestandteile der vorstehenden Zusammensetzungen addiert sich jeweils auf 100 Gew.-%.

Besonders bevorzugte Zusammensetzungen für die Reinigungsanwendungen sind erfindungsgemäße Zusammensetzungen aus Di-fluormethoxy-2,2,2-trifluorethan und einem Glykol, vorzugsweise Butylglykol.

Bevorzugte Zusammensetzungen, die für die erfindungsgemäße Verwendung zur Fixierung von Tonerpartikeln hervorragend geeignet sind, enthalten neben dem Hauptlösungmittel Difluormethoxy-2,2,2-trifluorethan ein Colösungsmittel aus der Gruppe der Ketone, vorzugsweise Aceton.

In einer besonders vorteilhaften Variante der Erfindung zeichnen sich die Zusammensetzungen aus Difluormethoxy-2,2,2-trifluorethan und Aceton durch ein azeotropartiges bzw. insbesondere azeotropes Verhalten aus. Solche azeotropartigen bzw. azeotropen Zusammensetzungen von Lösungsmitteln weisen eine Reihe von anwendungstechnischen Vorteilen auf. Einerseits sieden sie konstant bzw. im wesentlichen konstant (Schwankungen der Siedetemperatur um maximal etwa +/- 0,5 °C) und andererseits bleibt dabei auch die Zusammensetzung der Gemische konstant bzw. im wesentlichen konstant. Bei Verwendung azeotroper bzw. azeotropartiger Zusammensetzungen kommt es somit nicht zur Fraktionierung der Lösungsmittelbestandteile der Zusammensetzungen, wodurch unerwünschte Eigenschaftsveränderungen, wie z. B. veränderte Lösekraft, verminderte Inertheit gegenüber zu reinigenden Gegenständen oder erhöhte Entflammbarkeit bei Verwendung entzündlicher Colösungsmittel, vermieden werden. Darüber hinaus lassen sich azeotrope bzw. azeotropartige Zusammensetzungen nach Gebrauch leicht durch gewöhnliche Destillation reinigen und stehen somit in einfacher Weise für die Wiederverwendung zur Verfügung, ohne daß die Charakteristika der ursprünglichen Zusammensetzung verloren gehen.

Es wurde nun gefunden, daß einige erfindungsgemäße Zusammensetzungen aus E245 und Aceton sehr enge Siedebereiche aufweisen und sich somit zumindest azeotropartig verhalten. Diese speziellen azeotropartigen Zusammensetzungen enthalten 99,5 bis 95,0 Gew.-% Difluormethoxy-2,2,2-trifluorethan (E245) im Gemisch mit 0,5 bis 5,0 Gew.-% Aceton, wobei diese Zusammensetzungen in einem Bereich von 28,5 bis 33,0 °C sieden (Atmosphärendruck = 1 bar). Besonders günstig ist hierbei die azeotrope Zusammensetzung mit etwa 97,2 Gew.-% Difluormethoxy-2,2,2-trifluorethan und 2,8 Gew.-% Aceton, die einen Siedepunkt von etwa 28,5 °C bei Atmosphärendruck aufweist.

Die erfindungsgemäßen azeotropen bzw. azeotropartigen Zusammensetzungen aus Difluormethoxy-2,2,2-trifluorethan und Aceton besitzen günstige Löseeigenschaften. Hierdurch sind sie außer als Lösungsmittel in Fixiereinrichtungen von Druck- und Kopiergeräten auch für weitere Anwendungszwecke, z. B. als Lösungsmittel, geeignet.

Die erfindungsgemäßen Zusammensetzungen aus E245 und Aceton sind bei Raumtemperatur klare Lösungen, denen an sich bekannte Additive zugesetzt werden können (hierdurch wird das relative Verhältnis von Difluormethoxy-2,2,2-trifluorethan zu Aceton, welches durch die obigen Gew.-%-Angaben festgelegt ist, nicht verändert). Ebenso können auch den anderen erfindungsgemäßen Zusammensetzungen aus E245 und einem Colösungsmittel aus der Gruppe der Ketone, Ester, CKW und Glykole sowie auch den Zusammensetzungen aus E245 und niederen Alkoholen ansich bekannte Additive zugesetzt werden (das relative Verhältnis von E245 zum Colösungsmittel wird hier ebenfalls beibehalten). Die Additive werden nachfolgend für alle Zusammensetzungen gemeinsam beschrieben.

Eine Gruppe an sich bekannter Additive sind Stabilisatoren. Unter dieser Gruppe werden solche Verbindungen zusammengefaßt, die eine unerwünschte Reaktion von Bestandteilen der Zusammensetzung untereinander oder mit anderen Reaktionspartnern, wie beispielsweise Luftsauerstoff, Metall, Wasser usw., verhindern. Bekannte Stabilisatoren sind beispielsweise Nitroalkane, insbesondere Nitromethan, Nitroethan, Alkylenoxide, insbesondere Butylenoxid, oder verzweigte Alkinole wie z. B. 2-Methyl-butin-(3)-ol-(2). Diese Stabilisatoren können allein oder miteinander in Kombination eingesetzt sein, wobei Mengen von 0,01 bis 5 Gew.-%, vorzugsweise von 0,05 bis 1 Gew.-%, bezogen auf das Gesamtgemisch gut geeignet sind.

Eine weitere Gruppe von Additiven umfaßt an sich bekannte Verbindungen aus der Gruppe der Korrosionsinhibitoren, nicht-ionische oder ionische Emulgatoren, Farbstoffe etc.

Die erfindungsgemäßen Zusammensetzungen haben, insbesondere im Falle der Glykole als Colösungsmittel, auch Anwendungsmöglichkeiten auf dem Sektor der Reinigung und/oder Dampfentfettung. Bei diesen ansich bekannten Verfahren wird der zu reinigende Gegenstand in einer oder mehreren Stufen in flüssiges und/oder dampfförmiges Reinigungsgemisch getaucht oder mit flüssigem Reinigungsgemisch besprüht. Die Reinigungswirkung kann in bekannten Verfahren durch Anwendung bei Siedetemperatur und/oder Ultraschall und/oder Rühren gesteigert werden. Ebenso ist eine Verbesserung der Reinigungswirkung durch mechanische Einwirkung, wie z. B. Bürsten, bekannt.

Beispielsweise verwendet die elektronische Industrie für Lötverfahren vorherrschend organische Harzflußmittel, deren Überschüsse nach dem Lötvorgang von Leiterplatten entfernt werden müssen. Dieses erfolgt mit organischen Lösungsmitteln, die mit den Leiterplatten und den elektronischen Teilen verträglich sind, d. h. das Lösungsmittel darf nicht mit diesen reagieren. Die zu entfernenden Harzflußmittel sind Gemische polarer und nichtpolarer Verbindungen und enthalten oftmals zusätzlich spezielle Aktivatoren. Difluormethoxy-2,2,2-trifluorethan allein ist zur Entfernung der polaren Komponenten der Harze nicht wirksam. Auch ist es allein nicht in der Lage, insbesondere spezielle hochaktivatorhaltige Flußmittel vollständig zu entfernen. Überraschenderweise können aber die erfindungsgemäßen Zusammensetzungen aus E245 und Glykolen, z. B. Butylglykol, sowohl die polaren als auch die nichtpolaren Komponenten entfernen und sind daher als Entfernungsmittel für Harzflußmittel, insbesondere für solche mit hohem Aktivatorgehalt, auf breiter Basis wirksam.

So lassen sich unbestückte und bestückte (insbesondere auch SMD-bestückte) Leiterplatten auch bei Verwendung von Flußmitteln mit hohem Aktivatoranteil problemlos mit den erfindungsgemäßen Zusammensetzungen aus E245 und Butylglykol (2-Butoxy-ethanol) reinigen, ohne daß es zu den bei Einsatz der üblichen Reinigungsmittel gefürchteten "weißen Belägen" kommt.

Die neuen azeotropartigen und azeotropen Zusammensetzungen gemäß der Erfindung, insbesondere mit Aceton als Colösungsmittel, sind besonders günstige Systeme für die Fixierung von Toner auf Aufzeichnungsträgern in Druck- und Kopiergeräten (mit Ausnahme der Zusammensetzungen mit Glykolen als Colösungsmittel). Die hierfür verwendeten erfindungsgemäßen Zusammensetzungen können auch Stabilisatoren enthalten. Die erfindungsgemäßen Zusammensetzungen, insbesondere die azeotropartigen oder azeotropen Zusammensetzungen aus E245 und Aceton, lösen insbesondere Trockentoner auf Polystyrolbasis sehr gut an. Solche Toner können z. B. eine nichtvernetzte Polymermatrix aufweisen; z. B. können solche Toner eine Polymermatrix aus Polystyrol und Methacrylaten besitzen. Die erfindungsgemäßen Zusammensetzungen, insbesondere aus E245 und Aceton, eignen sich hervorragend für die Verwendung zur Tonerfixierung, insbesondere in modernen Laserdruckern. Sie eignen sich auch bei hohem Einfärbungsgrad des Aufzeichnungsträgers und sind insbesondere auch mit allen gängigen Papiersorten gut verträglich. Probleme einer Unterfixierung, wie diese bei Fixierlösungsmitteln des Standes der Technik häufig beoachtet werden, sind nicht zu befürchten. Ferner besitzen die erfindungsgemäßen, insbesondere die azeotropartigen und azeotropen Zusammensetzungen aus E245 und Aceton - ebenso auch die erfindungsgemäß verwendeten Zusammensetzungen aus E245 und C1- bis C3-Alkoholen bzw. aus E245 und CKWs - sehr gute toneranlösende Eigenschaften, insbesondere für Trockentoner auf Polystyrolbasis. Die erfindungsgemäßen bzw. erfindungsgemäß verwendeten Zusammensetzungen vermögen einerseits die Tonerpartikel sehr gut anzulösen ohne aber andererseits zu einem unerwünschten Zerfließen des Toners auf dem Aufzeichnungsträger zu führen. Hierdurch ist es möglich, gut haftende und nicht verwischende Bilder mit hoher Auflösung bzw. Schärfe auf dem Aufzeichnungsträger zu erzeugen.

Die erfindungsgemäßen Zusammensetzungen mit E245, die spezielle Colösungsmittel, z. B. Glykole enthalten, gewährleisten in hohem Maße den hohen Reinheitsgrad, der in speziellen Einsatzgebieten, z. B. bei der Reinigung von Bauteilen und Leiterplatten in der elektronischen Industrie, erforderlich ist. In ihren Eigenschaften stehen sie den bisher im Stand der Technik bekannten Zusammensetzungen mit vollhalogenierten Fluorchlorkohlenwasserstoffen nicht nach.

Ein weiterer Vorteil der erfindungsgemäßen Zusammensetzungen besteht in ihrem günstigen Flammpunktsverhalten, welches sich z. B. über die Methode "geschlossener Tiegel" prüfen läßt. Durch die erfindungsgemäßen Zusammensetzungen werden daher auf vielen Anwendungsgebieten neue Problemlösungen ermöglicht. Darüber hinaus erweist es sich als vorteilhaft, daß das eingesetzte Lösungsmittel Difluormethoxy-2,2,2-trifluorethan gegenüber vollhalogenierten Fluorchlorkohlenwasserstoffen deutlich leichtere Abbaubarkeit und niedriges globales Erwärmungspotential, also deutlich erhöhte Umweltverträglichkeit, zeigt.

Die Erfindung soll im Hinblick auf die Anwendungen nachfolgend noch beispielhaft näher erläutert werden, ohne sie jedoch in ihrem Umfang zu begrenzen. Sofern nicht anders angegeben, sind im folgenden % immer Gew.-%.

### Figurenbeschreibung:

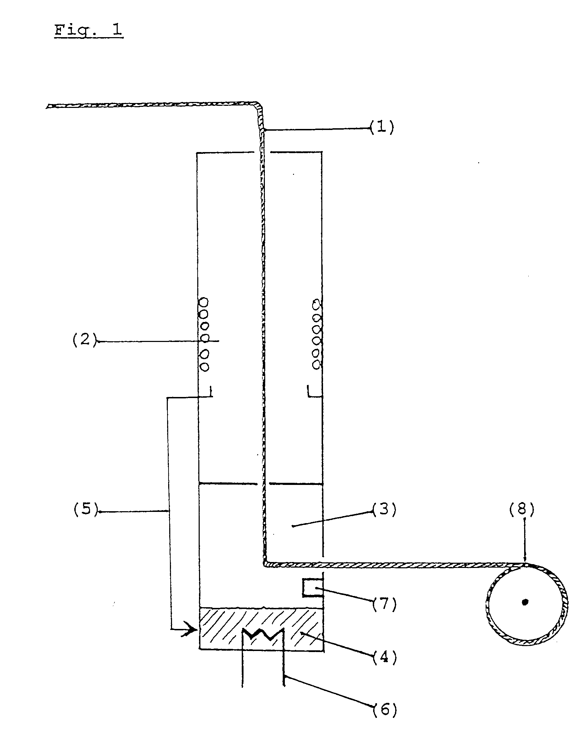

Fig. 1 Prinzipskizze einer Kaltfixierstation
   (1) = mit Toner beaufschlagte Papierbahn
   (2) = Kühlfalle
   (3) = Dampfkammer
   (4) = Lösungsmittelverdampfung
   (5) = Lösungsmittelrückführung
   (6) = Heizung
   (7) = Dampfsensor (Ultraschall) zur Messung der Dampfkonzentration
   (8) = Aufwicklung

### Toner-Fixierung

Die erfindungsgemäß für die Verwendung als Lösungsmittel zur Fixierung von Tonerpartikeln vorgeschlagenen Zusammensetzungen können mit einer Kaltfixierstation der in Fig. 1 angegebenen Prinzipdarstellung auf ihre prinzipielle Eignung zur Fixierung geprüft werden. Hierzu wird eine Papierbahn mit einem Toner auf Basis von Polystyrol beaufschlagt, z. B. in Form eines Musters oder einer Zahlen- bzw. Buchstabenfolge (bspw. Zeichenfolge einer bestimmten Schrifttype). Diese Beaufschlagung kann in ansich bekannter Weise geschehen, z. B. indem nach elektrostatischem Prinzip gebildete Ladungsbilder zunächst in einer Entwicklerstation mit Hilfe von Tonerpulver zu "Tonerbildern" entwickelt werden und diese Tonerbilder danach in einer Umdruckstation auf einen Aufzeichnungsträger (z. B. Papierbahn) übertragen werden. Diese Tonerbilder haften locker auf dem Aufzeichnungsträger und müssen anschließend unter Anlösen in den Aufzeichnungsträger "eingeschmolzen" werden, um eine verwischfreie Fixierung zu gewährleisten. Das Einschmelzen des Toners in die Papierbahn (= Aufzeichnungsträger) geschieht mit Hilfe des Lösungsmitteldampfes (E245 oder Gemische von E245 mit einem der hierfür beschriebenen Colösungsmittel in azeotropartiger bzw. azeotroper Zusammensetzung). Dazu wird ein Lösungsmitteldampf erzeugt, der geeignet ist, den Toner auf dem Aufzeichnungsträger anzulösen, wodurch der Toner dann in ausreichendem Maße in den Aufzeichnungsträger eindringen kann. Das mit dem Tonerbild versehene Aufzeichnungsmaterial wird hierzu in eine Fixiereinrichtung z. B. gemäß Fig. 1 eingeführt, in der Lösungsmitteldampf dann darauf einwirken kann. Eine solche Fixiervorrichtung besteht z. B. aus einem Gehäuse mit einer Führungseinrichtung für den Aufzeichnungsträger, die dazu dient, den Aufzeichnungsträger durch das Gehäuse hindurchzuführen. Ferner enthält eine Fixiervorrichtung eine Bedampfungsstelle, in der die Lösungmitteldämpfe möglichst austrittsfrei vorgehalten werden können. Eintritts- und Austrittsstellen der Fixiereinrichtung sind daher mit Kältefallen und Dichtungen versehen. Die Verdampfung bzw. Fixiermittelkonzentration wird durch an sich übliche Steuermechanismen geregelt.

Die erfindungsgemäß verwendeten Lösungsmitteldämpfe auf Basis von E245 zeigen gute toneranlösende Eigenschaften. Der Toner kann jeweils gut im Aufzeichnungsmaterial "eingeschmolzen" (fixiert) werden, ohne ein Verfließen der Ränder. Es lassen sich gut fixierte (verwischfreie) Abbildungen mit guter Schärfe auf den Aufzeichnungsträgern herstellen.

### Reinigung von Leiterplatten

In einer handelsüblichen 3-Kammer-Reinigungsanlage wurden Reinigungsversuche mit Leiterplatten, die sowohl mit üblichen halogenhaltigen Lötflußmitteln als auch mit stark aktivatorhaltigen Lötflußmitteln verunreinigt waren, vorgenommen. Sowohl die Verunreinigungen durch übliche halogenhaltige als auch durch stark aktivatorhaltige Lötflußmittel konnten von den Leiterplatten mit hervorragenden Reinigungsergebnissen entfernt werden. Die Reinigungszusammensetzungen, Reinigungsbedingungen und Reinigungsergebnisse sind in der Tabelle 1 wiedergegeben.

**Tabelle 1**

| **Nr.** | **Zusammensetzungen für Bad 1** | **Reinigungsbedingungen** | **Ergebnis** |
|---|---|---|---|
| 1 | E245 / Butylglykol: 90,0 % / 10,0 % | 3-Bad: | |
| | | 1) 3 Min. Ultraschall | ++ |
| | | 2) 1 Min. Ultraschall | |
| | | 3) 1 Min. Dampfentfettung | |
| | | (in Bad 2 und 3: E245) | |
| 2 | E245 / Butylglykol: 95,0 % / 5,0 % | 3-Bad: | |
| | | 1) 3 Min. Ultraschall | ++ |
| | | 2) 1 Min. Ultraschall | |
| | | 3) 1 Min. Dampfentfettung | |
| | | (in Bad 2 und 3: E245) | |

In den in der Spalte "Ergebnis" mit "++" gekennzeichneten Fällen ist eine sehr gute Reinigungswirkung erzielt worden und es kam nicht zur Bildung "weißer Beläge". Es ist deutlich sichtbar, daß die erfindungsgemäßen Zusammensetzungen hervorragende Reinigungsleistungen zeigen.

## Patentansprüche

1. Verwendung von Zusammensetzungen, enthaltend Difluormethoxy-2,2,2-trifluorethan (E245) und ggf. ein Colösungsmittel, als Lösungsmittel zur Fixierung von Tonern, vorzugsweise von Tonern auf Basis von Polystyrol, in der Fixiervorrichtung von Druck- und Kopiergeräten, insbesondere von Laserdruckern.

2. Verwendung nach Anspruch 1, dadurch gekennzeichnet, daß Difluormethoxy-2,2,2-trifluorethan (E245) in azeotropartiger Zusammensetzung mit einem Colösungsmittel eingesetzt wird.

3. Verwendung nach Anspruch 1 oder 2, dadurch gekennzeichnet, daß das Colösungsmittel ausgewählt ist aus der Gruppe der niederen Alkohole, Ketone, Ester und chlorierten Kohlenwasserstoffe.

4. Verwendung nach Anspruch 3, dadurch gekennzeichnet, daß das Colösungsmittel ein Keton, vorzugsweise Aceton, ist.

5. Verwendung nach einem der Ansprüche 2 bis 4, dadurch gekennzeichnet, daß das Colösungsmittel in einer Menge von 0,2 bis 5,0 Gew.-%, insbesondere 0,3 bis 5,0 Gew.-%, und das Hauptlösungsmittel E245 demgemäß in einer Menge von 99,8 bis 95,0 Gew.-% bzw. 99,7 bis 95,0 Gew.-% in den Zusammensetzungen enthalten ist, wobei die Summe der Bestandteile 100 Gew.-% ist.

6. Verwendung nach einem der vorhergehenden Ansprüche, dadurch gekennzeichnet, daß eine azeotrope binäre Zusammensetzung aus 97,2 Gew.-% E245 und aus 2,8 Gew.-% Aceton als Colösungsmittel eingesetzt wird.

7. Verfahren zum Fixieren von auf einem Aufzeichnungsträger aufgebrachten auf Polystyrolbasis aufgebauten Toner, wobei ein in der Fixiervorrichtung von Druck- oder Kopiergeräten enthaltenes, gegebenenfalls stabilisatorhaltiges, Lösungsmittel aus einem azeotropartigen oder azeotropen Gemisch von reinen Flüssigkeiten, von denen mindestens eine toneranlösende Eigenschaften hat, verdampft wird und in diesem Zustand auf den Toner einwirkt und diesen anlöst, dadurch gekennzeichnet, daß als Lösungsmittel in der Fixiervorrichtung eine Zusammensetzung eingesetzt wird, die als Lösungsmittel Difluormethoxy-2,2,2-trifluorethan (E245) und ggf. ein Colösungsmittel aus der Gruppe der niederen Alkohole, Ketone, Ester und chlorierten Kohlenwasserstoffe enthält.

8. Zusammensetzungen aus Difluormethoxy-2,2,2-trifluorethan (E245) und einem Colösungsmittel aus der Gruppe der Ketone, Ester, chlorierten Kohlenwasserstoffe und Glykole.

9. Zusammensetzungen nach Anspruch 8, gekennzeichnet durch einen Gehalt an 99,8 bis 50,0 Gew.-% Difluormethoxy-2,2,2-trifluorethan (E245) und 0,2 bis 50,0 Gew.-% des Colösungsmittels aus der Gruppe der Ketone, Ester, chlorierten Kohlenwasserstoffe und Glykole, wobei die Summe der Bestandteile 100 Gew.-% ist.

10. Zusammensetzungen nach Anspruch 9, gekennzeichnet durch einen Gehalt an 99,8 bis 90,0 Gew.-% Difluormethoxy-2,2,2-trifluorethan (E245) und 0,2 bis 10,0 Gew.-% des Colösungsmittels aus der Gruppe Ketone, Ester, chlorierten Kohlenwasserstoffe und Glykole.

11. Zusammensetzungen nach Anspruch 10, dadurch gekennzeichnet, daß sie Difluormethoxy-2,2,2-trifluorethan (E245) in einer Menge von 99,8 bis 95,0 Gew.-% und das Colösungsmittel aus der Gruppe der Ketone, Ester, chlorierten Kohlenwasserstoffe und Glykole in einer Menge von 0,2 bis 5,0 Gew.-% enthalten.

12. Zusammensetzungen nach einem der Ansprüche 8 bis 11, dadurch gekennzeichnet, daß das Colösungsmittel ein Keton, vorzugsweise Aceton, ist.

13. Zusammensetzungen nach einem der Ansprüche 8 bis 12, gekennzeichnet durch eine azeotropartige Zusammensetzung von 99,5 bis 95,0 Gew.-% Difluormethoxy-2,2,2-trifluorethan (E245) im Gemisch mit 0,5 bis 5,0 Gew.-% Aceton.

14. Zusammensetzung nach Anspruch 13, gekennzeichnet durch eine azeotrope Zusammensetzung von etwa 97,2 Gew.-% Difluormethoxy-2,2,2-trifluorethan (E245) und etwa 2,8 Gew.-% Aceton.

15. Zusammensetzungen gemäß einem der Ansprüche 8 bis 14, gekennzeichnet durch einen Gehalt an Stabilisator von 0,01 bis 5 Gew.-%, vorzugsweise 0,05 bis 1 Gew.-%, bezogen auf das Gesamtgemisch, vorzugsweise aus der Gruppe Nitroalkan, Alkylenoxid und/oder Alkinol.

16. Verfahren zum Reinigen von Oberflächen, dadurch gekennzeichnet, daß man die Oberflächen mit Zusammensetzungen nach einem der Ansprüche 8 bis 15 behandelt.

17. Verfahren nach Anspruch 16, dadurch gekennzeichnet, daß die zu reinigenden Oberflächen mit Lötflußmittel oder Lötflußmittelrückständen verunreingte gedruckte Leiterplatten sind.

18. Verfahren nach einem der Ansprüche 16 und 17, dadurch gekennzeichnet, daß man die Oberflächen mit einer Zusammensetzung aus Difluormethoxy-2,2,2-trifluorethan (E245) und aus einem Glykol als Colösungsmittel, vorzugsweise Butylglykol, behandelt.
